# NEUE EUROPÄISCHE PATENTSCHRIFT

(11) **EP 2 167 169 B2**
(45) Veröffentlichungstag und Bekanntmachung des Hinweises auf die Entscheidung über den Einspruch: **09.03.2016**
(45) Hinweis auf die Patenterteilung: 23.05.2012
(21) Anmeldenummer: 08758924.8
(22) Anmeldetag: 31.05.2008
(51) Int. Cl.: A61M 5/28, A61M 5/31

(54) **ZWEIKAMMERKARPULE MIT AUFSATZ**
DOUBLE CHAMBER CARTRIDGES WITH ATTACHMENT
CARPULE À DOUBLE CHAMBRE DOTÉE D'UN CHAPITEAU

(30) Priorität: 14.06.2007 EP 07011687
(43) Veröffentlichungstag der Anmeldung: 31.03.2010
(73) Patentinhaber: Sanofi-Aventis Deutschland GmbH, 65929 Frankfurt am Main (DE)
(72) Erfinder: SEIFERLEIN, Werner, 65926 Frankfurt am Main (DE); MÖCKEL, Jörn, 65926 Frankfurt am Main (DE)
(74) Vertreter: Finger, Catrin
(86) Internationale Anmeldenummer: PCT/EP2008/004355
(87) Internationale Veröffentlichungsnummer: WO 2008/151736

(56) Entgegenhaltungen:
- EP-A- 0 397 977
- EP-A- 0 568 321
- EP-A- 1 066 847
- EP-A- 1 093 826
- EP-A- 1 287 841
- EP-A1- 1 728 560
- EP-A2- 0 520 618
- EP-B1- 0 248 210
- EP-B1- 0 302 248
- WO-A1-02/39920
- WO-A1-92/01485
- DE-A1- 2 163 668
- DE-A1- 2 439 312
- DE-A1-102005 038 458
- DE-A1-102005 038 495
- FR-A1- 2 178 019
- GB-A- 705 392
- GB-A- 705 392
- US-A- 2 880 725
- US-A- 3 102 539
- US-A- 4 226 236
- US-A- 4 226 236
- US-A- 4 254 768
- US-A- 5 069 670
- US-A1- 2003 199 816
- US-B1- 6 261 094

## Beschreibung

Zweikammerkarpulen werden in der Medizin zur Verabreichung von Präparaten eingesetzt, die aus zwei Komponenten bestehen. Es gibt dabei zwei Möglichkeiten, die Komponenten zu kombinieren, nämlich die Kombination flüssig/flüssig und die Kombination fest/flüssig. Zweikammerkarpulen haben den Vorteil, dass das Mischen der beiden Komponenten ohne Umfüllen in ein anderes Behältnis erfolgen kann und dass aus dem Behältnis heraus dann direkt die Verabreichung erfolgen kann. Zweikammerkarpulen können in dafür vorgesehene wiederverwendbare Spritzen- oder Pen-Systeme eingesetzt werden.

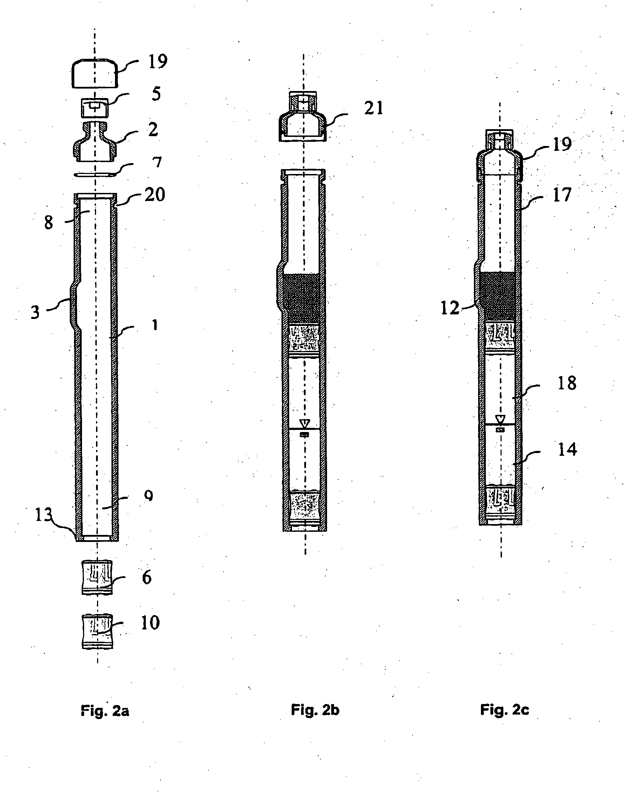

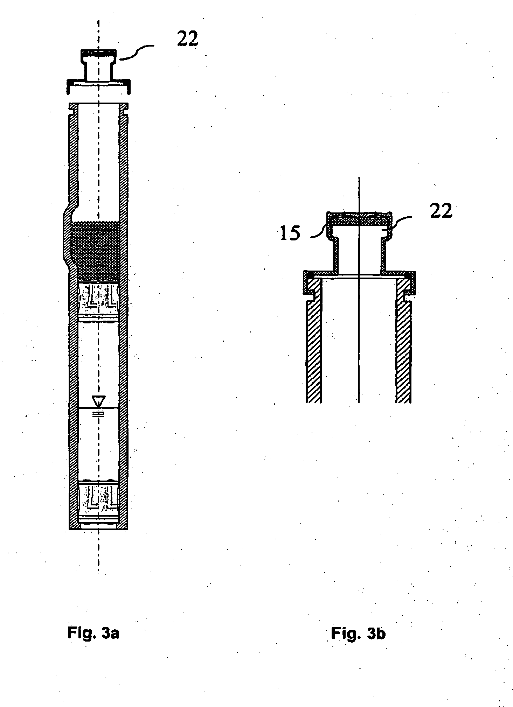

DE 4445969, EP 718002 und US 5,788,670 beschreiben Spritzenzylinder von Zweikammerspritzen für zwei zu applizierende Komponenten, wobei ein erster Teilzylinder, vorzugsweise der kopfseitige Teilzylinder, einen zu lyophilisierenden Wirkstoff enthält, und ein zweiter Teilzylinder, vorzugsweise der kolbenseitige Teilzylinder, eine flüssige Komponente enthält. Während der Verabreichung vermischt sich der pulverförmige Wirkstoff mit der flüssigen Komponente über einen Bypass. Der nadelseitige Teilzylinder enthält den Bypass und ist mit einem Stopfen am proximalen Ende verschlossen. Der kolbenseitige Teilzylinder ist mit einem Stopfen am proximalen, kolbenseitigen Ende (Fig. 2) und optional mit einem weiteren Stopfen am distalen Ende (Fig. 3) verschlossen. Beide Teilzylinder werden mittels stoffschlüssig (durch Verschweißen) oder kraftschlüssig (durch Verkleben) miteinander verbunden. Die beschriebenen Spritzenzylinder besitzen einen Bypass, der vorzugsweise so ausgeformt ist, dass er den Durchmesser des Spritzenzylinders nicht vergrößert. Das Material des vorderen Teilzylinders ist vorzugsweise Kunststoff. In dem Verfahren zur Befüllung des Spritzenzylinders ist der Spritzenkopf des ersten Teilzylinders durch ein sogenanntes tip cap verschlossen, d.h. durch einen losen Verschluss, durch den nach dem Befüllen mit dem zu lyophilisierenden Wirkstoff bei der anschließenden Gefriertrocknung das umgebende Vakuum an das Innere des Spritzenzylinders weitergegeben werden kann, und das erst nach dem Gefriertrocknungsschritt fest verschlossen wird. Die getrennte Abfüllung in einen ersten und einen zweiten Teilzylinder vermeidet eine "Cross Contamination" und ermöglicht eine optimale Auslastung des Prozessraums des Lyophilisators.

Die europäische Patentanmeldung EP 520618 beschreibt eine vorgefüllte Spritze bestehend aus zwei Teilzylindern, wobei der erste Teilzylinder, vorzugsweise der kopfseitige Teilzylinder, der die Nadel bzw. den Auslass beinhaltet, ein lyophilisiertes, pulverförmiges Medikament enthält, und der zweite Teilzylinder, vorzugsweise der kolbenseitige Teilzylinder, eine zweite, flüssige Komponente enthält. Während der Verabreichung vermischt sich das pulverförmige Medikament mit der flüssigen Komponente über einen Bypass. Der nadelseitige Teilzylinder enthält den Bypass und ist mit einem Stopfen am proximalen Ende verschlossen. Der kolbenseitige Teilzylinder ist mit jeweils einem Stopfen am distalen und am proximalen Ende verschlossen. Beide Teilzylinder werden mittels der Stopfen getrennt versiegelt und über Flansche miteinander verbunden, wobei der proximale Stopfen des kopfseitigen Teilzylinders und der distale Stopfen des kolbenseitigen Teilzylinders nach dem Zusammenfügen formschlüssig miteinander verbunden sind und die Gesamtlänge beider Stopfen kürzer ist als die Länge des Bypasses.

GB 2010681 beschreibt eine Zweikammerspritze zur Verabreichung einer Flüssigkeit bestehend aus zwei Teilzylindern, wobei der erste Teilzylinder als Nadelhalter ausgebildet ist und einen Kanal zum Auslass beinhaltet und der zweite, kolbenseitige Teilzylinder Flüssigkeit enthält. Der erste Teilzylinder enthält keine zu verabreichende Substanz. Während der Verabreichung wird ein Stopfen, mit dem der kolbenseitige Teilzylinder am distalen Ende verschlossen ist, in Richtung Spritzenkopf bewegt, wodurch die Flüssigkeit über den Kanal des ersten Teilzylinders appliziert werden kann.

FR 1099362 beschreibt eine Zweikammerspritzen bestehend aus zwei Teilzylindern, wobei der erste, kopfseitige Teilzylinder, ein steriles Pulver oder eine sterile Flüssigkeit enthält, und der zweite, kolbenseitige Teilzylinder, eine Flüssigkeit enthält. Während der Verabreichung vermischt sich das sterile Pulver bzw. die sterile Flüssigkeit des ersten Teilzylinders mit der Flüssigkeit des zweiten Teilzylinders über einen Bypass im ersten, nadelseitigen Teilzylinder. Der kolbenseitige Teilzylinder ist mit einem Stopfen am distalen und einem Kolben am proximalen Ende verschlossen. Beide Teilzylinder werden über Flansche oder mittels eines weiteren (Über-)Zylinders, dessen Innendurchmesser dem Außendurchmesser der beiden Teilzylinder entspricht, durch Verkleben oder Verschweißen miteinander verbunden.

Die Patentschrift EP 0 397 977 A offenbart eine Zweikammerkarpule bestehend aus einem Zylinder und einem Aufsatz, wobei der Aufsatz mit radial verlaufenden Durchgangsbohrungen versehen ist, sodass das bei einer Lyphilisierung frei werdende Lösungsmittel über die Durchgangsbohrungen austreten kann.

Die Spritzensysteme im Stand der Technik haben den Nachteil, dass diese, bedingt durch das mit Nadelhalterung ausgeführte distale Ende nicht zum Einsetzen in eine Spritzenapplikation (Pen u.a.) geeignet sind und/oder der feste Wirkstoff zunächst durch eine vergleichsweise kleine Öffnung als Lösung oder Suspension eingefüllt werden muss und anschließend im nadelseitigen Teilzylinder lyophilisiert werden muss, um eine feste, pulverförmige Komponente zu erhalten. Zudem ist im Stand der Technik keine Lösung genannt, nach der der beim Zusammenfügen der beiden Teilzylinder entstehende Druck kompensiert wird.

Aufgabe der vorliegenden Erfindung ist es daher, eine verbesserte Zweikammerkarpule und ein verbessertes Verfahren zur Herstellung von Zweikammerkarpulen sowie zum Befüllen von Zweikammerkarpulen bereit zu stellen.

Die vorliegende Erfindung betrifft eine Zweikammerkarpule gemäß des Anspruchs 1.

Vorteil hafte Ausgestaltungen der zweikammerhaspule gehen aus den Unteransprüchen hervor.

Zwischen Endstopfen und Zwischenstopfen wird eine Kammer (18) gebildet und zwischen Zwischenstopfen und Aufsatz eine Kammer (17).

Der Aufsatz bildet den Abschluss des Zylinders (1) und wird oberhalb (distal) des Bypasses aufgesetzt. Vorzugsweise wird in der kopfseitigen Kammer (17) eine trockene Wirkstoffzubereitung (12) gefüllt. Stopfen (6) kann sich einwandfrei über der Nahtstelle bewegen. Zylinder und Verschluss sind dicht miteinander verbunden.

Der Zylinder kann aus einem oder aus zwei Teilzylindern bestehen. Der Nadelhalter ist eine Vorrichtung zur Aufnahme einer Vorrichtung, an der eine Nadel befestigt ist.

Die vordere Kammer (17), die im montierten Zustand zwischen dem Zwischenstopfen (6) und dem Aufsatz gebildet wird, enthält eine feste oder eine flüssige Komponente, vorzugsweise eine feste Komponente, besonders bevorzugt ein Lyophilisat oder ein Pulver. Die hintere Kammer (18), die im montierten Zustand zwischen Endstopfen (10) und Zwischenstopfen (6) gebildet wird, enthält eine flüssige Komponente. Die feste oder flüssige Komponente der vorderen Kammer und die flüssige Komponente der hinteren Kammer bilden das zu verabreichende Medikament.

Das proximale Ende des Zylinders (1) weißt vorzugsweise einen Anschlag (13) auf. Der Anschlag verhindert ein Verrutschen des Endstopfens (10) durch den beim Zusammenfügen entstehenden Druck in Richtung proximales Ende. Der Anschlag ist so ausgeformt, dass der Kolben eines Applikationssystems Kraft auf den Endstopfen übertragen kann.

"Proximal" bedeutet das Ende eines Bauteils, das im zusammengebauten Zustand zum Endstopfen (10) weißt, also zum Kolben bzw. zum den Kolben betätigenden Finger des Verabreichenden, wenn die Zweikammerkarpule in ein Pen-System eingebaut ist und ein am Endstopfen (10) angreifender Kolben des Applikationssystems durch den Daumen des Verabreichenden betätigt wird. Das proximale Ende des Aufsatzes ist das Ende des Kopfes, das im zusammengebauten Zustand mit dem distalen Ende des Zylinders verbunden ist.

"Distal" bedeutet das Ende eines Bauteils, das im zusammengebauten Zustand zum zur Auslassöffnung weißt.

Das "kopfseitige Ende der Zweikammerkarpule" ist das Ende der Zweikammerkarpule, das den Kopf der Zweikammerkarpule bildet, das also die Austassöffnung beinhaltet.

Das "kolbenseitige Ende der Zweikammerkarpule" ist das Ende der Zweikammerkarpule, an dem sich der Endstopfen (10) befindet und an dem in einem Applikations-System der Kolben angreift.

Stopfen und Dichtungselemente sind unabhängig voneinander aus elastischem Material, beispielsweise Kautschuk, vorzugsweise Brombutyl-, Chlorbutyl- oder Fluorbutyl-Kautschuk. Optional sind die Stopfen mit PTFE beschichtet. Die Stopfen sind von vorzugsweise zylindrischer Grundform, aber auch anderer Grundformen entsprechend der inneren Ausformung der Teilzylinder sind möglich. Die Stopfen haben sowohl eine Dichtungs- als auch eine Verschlussfunktion, beispielsweise verschließt und dichtet Zwischenstopfen (6) die Kammer (18). Die Dichtfunktion wird vorzugsweise durch eine oder mehrere lamellenartige Ausformungen der zylindrischen Grundform sichergestellt.

"Dicht" meint die Undurchlässigkeit gegenüber Feststoffen, Flüssigkeiten, Gasen, sowie gegenüber Keimen.

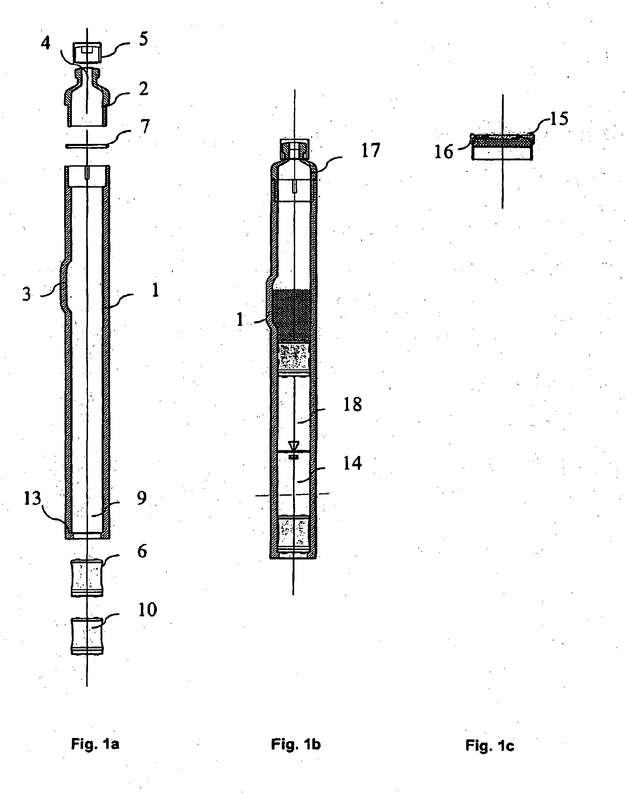

Der Bypass (3) ist eine Öffnung, die es bei Verwendung der Zweikammerkarpule ermöglicht, dass eine flüssige Komponente (14) während der Verabreichung des Medikaments unter Umgehung des Zwischenstopfens (6) aus Kammer (18) in Kammer (17) einströmen kann. Der Bypass (3) kann erzeugt werden durch einen oder mehrere Kanäle, die sich im Wandmaterial des Teilzylinders (1) befinden, d.h. in das Wandmaterial eingelassen bzw. eingearbeitet sind. Der Bypass kann auch durch entsprechende Ausformung des Wandmaterials nach innen (nicht abgebildet) oder nach außen (wie beispielsweise in Figur 1a abgebildet) geformt werden. Die Anordnung kann axial oder von der axialen Richtung radial abweichend ausgeführt sein.

Zylinder und Aufsatz sind unabhängig voneinander aus Glas, Kunststoff, Metall oder anderen Werkstoffen geformt, bevorzugt transparente Werkstoffe wie Glas oder Kunststoff.

Kunststoffe sind bevorzugt Polycarbonate, Polyester, Cyclo-Olefin-Copolymere (COC) oder Cyclic Olefin Polymere (COP).

Das Medikament enthält einen oder mehrere pharmazeutisch wirksame Bestandteile ausgewählt aus der Gruppe (i) eine niedermolekulare Verbindung (mit einem Molekülgewicht bis zu 1500 Da), (ii) ein Peptid, (iii) ein Protein, (iv) DNA, (v) RNA, (vi) Antikörper, (vii) Enzym und (viii) Oligonukleotid,

vorzugsweise enthaltend mindestens ein Peptid, bevorzugt ein Peptid zur Behandlung von Diabetes mellitus oder Komplikationen von Diabetes mellitus wie beispielsweise Diabetesche Retinopathie,

besonders bevorzugt ausgewählt aus der Gruppe Humaninsulin, ein Humaninsulinanalogon, ein Humaninsulinderivat, Glucagon-Like Peptide-1 (GLP1), ein GLP1-Analogon, ein GLP1-Derivat, Exendin-3, Exendin-4, ein Exendin-3-Analogon, ein Exendin-4-Analogon, ein Exendin-3-Derivat oder ein Exendin-4-Derivat.

Insulinanaloga sind beispielsweise Gly(A21), Arg(B31), Arg(B32)- Humaninsulin; Lys(B3), Glu(B29)- Humaninsulin; Lys(B28), Pro(B29)- Humaninsulin; Asp(B28)- Humaninsulin; Humaninsulin, bei dem Prolin in der Position B28 substituiert wurde durch Asp, Lys, Leu, Val oder Ala und wo in Position B29 Lys durch Pro substituiert sein kann; Ala(B26)-Humaninsulin; Des(B28-B30)- Humaninsulin; Des(B27)- Humaninsulin und Des(B30)-Humaninsulin.

Insulinderivate sind beispielsweise B29-N-myristoyl-des(B30) Humaninsulin; B29-N-palmitoyl-des(B30) Humaninsulin; B29-N-myristoyl Humaninsulin; B29-N-palmitoyl Humaninsulin; B28-N-myristoyl LysB28ProB29 Humaninsulin; B28-N-palmitoyl-LysB28ProB29 Humaninsulin; B30-N-myristoyl-ThrB29LysB30 Humaninsulin; B30-N-palmitoyl- ThrB29LysB30 Humaninsulin; B29-N-(N-palmitoyl-Y-glutamyl)-des(B30) Humaninsulin; B29-N-(N-lithocholyl-Y-glutamyl)-des(B30) Humaninsulin; B29-N-(ω-carboxyheptadecanoyl)-des(B30) Humaninsulin und B29-N-(ω-carboxyheptadecanoyl) Humaninsulin.

Exendin-4 bedeutet vorzugsweise Exendin-4(1-39), ein Peptid mit der Sequenz H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4-Derivate sind beispielsweise ausgewählt aus der folgenden Gruppe von Verbindungen:
H-(Lys)₄-des Pro³⁶, des Pro³⁷ Exendin-4(1-39)-NH₂,
H-(Lys)₅-des Pro³⁶, des Pro³⁷ Exendin-4(1-39)-NH₂,
des Pro³⁶ [Asp²⁸] Exendin-4(1-39),
des Pro³⁶ [IsoAsp²⁸] Exendin-4(1-39),
des Pro³⁶ [Met(O)¹⁴, Asp²⁸] Exendin-4(1-39),
des Pro³⁶ [Met(O)¹⁴, IsoAsp²⁸] Exendin-4(1-39),
des Pro³⁶ [Trp(O₂)²⁵, Asp²⁸] Exendin-4(1-39),
des Pro³⁶ [Trp(O₂)²⁵, IsoAsp²⁸] Exendin-4(1-39),
des Pro³⁶ [Met(O)¹⁴ Trp(O₂)²⁵, Asp²⁸] Exendin-4(1-39),
des Pro³⁶ [Met(O)¹⁴ Trp(O₂)²⁵, IsoAsp²⁸] Exendin-4(1-39); oder

des Pro³⁶ [Asp²⁸] Exendin-4(1-39),
des Pro³⁶ [IsoAsp²⁸] Exendin-4(1-39),
des Pro³⁶ [Met(O)¹⁴, Asp²⁸] Exendin-4(1-39),
des Pro³⁶ [Met(O)¹⁴, IsoAsp²⁸] Exendin-4(1-39),
des Pro³⁶ [Trp(O₂)²⁵, Asp²⁸] Exendin-4(1-39),
des Pro³⁶ [Trp(O₂)²⁵, IsoAsp²⁸] Exendin-4(1-39),
des Pro³⁶ [Met(O)¹⁴ Trp(O₂)²⁵, Asp²⁸] Exendin-4(1-39),
des Pro³⁶ [Met(O)¹⁴ Trp(O₂)²⁵, IsoAsp²⁸] Exendin-4(1-39),
wobei die Gruppe -Lys₆-NH₂ mit dem C-Terminus des Exendin-4-Derivats verknüpft ist; oder
ein Exendin-4-Derivat der Sequenz
H-(Lys)₆-des Pro³⁶ [Asp²⁸] Exendin-4(1-39)-Lys₆-NH₂,
des Asp²⁸ Pro³⁶, Pro³⁷, Pro38Exendin-4(1-39)-NH₂,
H-(Lys)₆-des Pro³⁶, Pro³⁸ [Asp²⁸] Exendin-4(1-39)-NH₂,
H-Asn-(Glu)₅des Pro³⁶, Pro³⁷, Pro³⁸ [Asp²⁸] Exendin-4(1-39)-NH₂,
des Pro³⁶, Pro³⁷, Pro³⁸ [Asp²⁸] Exendin-4(1-39)-(Lys)₆-NH₂,
H-(Lys)₆-des Pro³⁶, Pro³⁷, Pro³⁸ [Asp²⁸] Exendin-4(1-39)-(Lys)₆-NH₂,
H-Asn-(Glu)₅-des Pro³⁶, Pro³⁷, Pro³⁸ [Asp²⁸] Exendin-4(1-39)-(Lys)₆-NH₂,
H-(Lys)₆-des Pro³⁶ [Trp(O₂)²⁵, Asp²⁸] Exendin-4(1-39)-Lys₆-NH₂,
H-des Asp²⁸ Pro³⁶, Pro³⁷, Pro³⁸ [Trp(O₂)²⁵] Exendin-4(1-39)-NH₂,
H-(Lys)₆-des Pro³⁶, pro³⁷, Pro³⁸ [TrP(O₂)²⁵, Asp²⁸] Exendin-4(1-39)-NH₂,
H-Asn-(Glu)₅-des Pro³⁶, Pro³⁷, Pro³⁸ [Trp(O₂)²⁵, Asp²⁸] Exendin-4(1-39)-NH₂,
des Pro³⁶, Pro³⁷, Pro³⁸ [Trp(O₂)²⁵, Asp²⁸] Exendin-4(1-39)-(Lys)₆-NH₂,
H-(Lys)₆-des Pro³⁶, Pro³⁷, Pro³⁸ [Trp(O₂)²⁵, Asp²⁸] Exendin-4(1-39)-(Lys)₆-NH₂,
H-Asn-(Glu)₅-des Pro³⁶, Pro³⁷, Pro³⁸ [Trp(O₂)²⁵ , Asp²⁸] Exendin-4(1-39)-(Lys)₆-NH₂,
H-(Lys)₆-des Pro³⁶ [Met(O)¹⁴, Asp²⁸] Exendin-4(1-39)-Lys₆-NH₂,
des Met(O)¹⁴ Asp²⁸ Pro³⁶, Pro³⁷, Pro³⁸ Exendin-4(1-39)-NH₂,
H-(Lys)₆-desPro³⁶, Pro³⁷, Pro³⁸ [Met(O)¹⁴, Asp²⁸] Exendin-4(1-39)-NH₂,
H-Asn-(Glu)₅-des Pro³⁶, Pro³⁷, Pro³⁸ [Met(O)¹⁴, Asp²⁸] Exendin-4(1-39)-NH₂,
des Pro³⁶, pro³⁷, Pro³⁸ [Met(O)¹⁴, Asp²⁸] Exendin-4(1-39)-(Lys)₆-NH₂,
H-(Lys)₆-des Pro³⁶, Pro³⁷, Pro³⁸ [Met(O)¹⁴, Asp²⁸] Exendin-4(1-39)-(Lys)₆-NH₂,
H-Asn-(Glu)₅ des Pro³⁶, Pro³⁷, Pro³⁸ [Met(O)¹⁴, Asp²⁸] Exendin-4(1-39)-(Lys)₆-NH₂,
H-Lys₆-des Pro³⁶, [Met(O)¹⁴, Trp(O₂)²⁵, Asp²⁸] Exendin-4(1-39)-Lys₆-NH₂,
H-des Asp²⁸ Pro³⁶, Pro³⁷, Pro³⁸ [Met(O)¹⁴, Trp(O₂)²⁵] Exendin-4(1-39)-NH₂,
H-(Lys)₆-des Pro³⁶, Pro³⁷, Pro³⁸ [Met(O)¹⁴, Asp²⁸] Exendin-4(1-39)-NH₂,
H-Asn-(Glu)₅-des Pro³⁶, Pro³⁷, Pro³⁸ [Met(O)¹⁴, Trp(O₂)²⁵, Asp²⁸] Exendin-4(1-39)-NH₂,
des Pro³⁶, Pro³⁷, Pro³⁸ [Met(O)¹⁴, Trp(O₂)²⁵, Asp²⁸] Exendin-4(1-39)-(Lys)₆-NH₂,
H-(Lys)₆-des Pro³⁶, Pro³⁷, Pro³⁸ [Met(O)¹⁴, Trp(O₂)²⁵, Asp²⁸] Exendin-4(S1-39)-(Lys)₆-NH₂,
H-Aₛₙ-(Glu)₅-des Pro³⁶, Pro³⁷, Pro³⁸ [Met(O)¹⁴, Trp(O₂)²⁵, Asp²⁸] Exendin-4(1-39)-(Lys)₆-NH₂;
oder ein pharmazeutisch akzeptables Salz oder Solvat von Exendin-4 oder eines der vorgenannten Exendin-4-Derivate.

Vorzugsweise ist der pharmazeutisch wirksame Bestandteil die feste Komponente in der Kammer des vorderen Teilzylinders, besonders bevorzugt ein Lyophilisat oder ein Pulver.

Pharmazeutisch akzeptable Salze sind beispielsweise Säureadditionssalze und basische Salze. Säureadditionssalze sind beispielsweise HCl- oder HBr-Additionssalze. Basische Salze sind beispielsweise Salze, in denen das Kation ausgewählt ist aus der Gruppe der Alkalisalze, beispielsweise Na⁺ oder K⁺, oder der Erdalkalisalze, beispielsweise Ca²⁺, oder Ammoniumionen N⁺(R₁)(R₂)(R₃)(R₄), wobei R₁ bis R₄ unabhängig voneinander bedeuten: Wasserstoff, C₁-C₆-Alkyl, C₂-C₆-Alkenyl, C₆-C₁₀-Aryl, oder C₆-C₁₀-Heteroaryl. Weitere Beispiels von pharmazeutisch akzeptablen Salze sind beschrieben in "Remington's Pharmaceutical Sciences" 17. Ed. Alfonso R. Gennaro (Ed.), Mark Publishing Company, Easton, Pa., U.S.A., 1985 und in Encyclopedia of Pharmaceutical Technology.

Aufsatz und Zylinder werden auf dem Fachmann bekannte Weise form- und/oder kraftschlüssig miteinander verbunden, beispielsweise durch Schraubverschluss, Steckverschluss, Bajonettverschluss, Schnappverschluss oder Klemmverschluss (in den Figuren dargestellt). Abhängig von der Wahl der Werkstoffe ist die Verbindung bevorzugt selbstdichtend oder wird beispielsweise durch Verkleben oder Verschweißen bewerkstelligt. Optional kann ein Dichtungselement eingesetzt werden.

In einer Ausführungsform ist der Zylinder (1) das aufnehmende Bauteil. Werden Kopf (2) und Zylinder (1) beispielsweise durch Schraubverschluss miteinander verbunden, weißt der Zylinder (1) am distalen Ende ein Innengewinde auf, und Kopf (2) am proximalen Ende ein entsprechendes Außengewinde.

In einer weiteren Ausführungsform ist Kopf (2) das aufnehmende Bauteil. Werden Kopf (2) und Zylinder (1) beispielsweise durch Schraubverschluss miteinander verbunden, weißt der Kopf (2) am proximalen Ende ein Innengewinde auf, und Zylinder (1) am distalen Ende ein entsprechendes Außengewinde.

In einer nicht erfindungsgemäßen Ausführungsform besteht der Verschluss aus einer integrierten Verschlusskappe, die mit und ohne Schulter ausgeformt sein kann. Bei einer Ausformung mit Schulter enthält der Aufsatz bevorzugt ein Dichtelement zum Abdichten des distalen Ende des integrierten Verschlusses und ein weiteres Dichtelement zum Abdichten der Verbindung zwischen integriertem Verschluss und Zylinder. Das Dichtungselement ist bei zylindrischem Querschnitt von Zylinder und Verschluss ein Dichtring.

Nicht erfindungsgemäß besteht der Aufsatz einer einstückigen, integrierten Verschlusskappe (22) mit einer distalen Öffnung zum Aufsetzen auf den Zylinder (1) und ferner beinhaltend ein Dichtelement zum Abdichten des distalen Endes der integrierten Verschlusskappe (22) und optional (bevorzugt) ein weiteres Dichtelement (7) zum Abdichten der Verbindung zwischen integrierter Verschlusskappe (22) und Zylinder (1). Die integrierte Verschlusskappe (22) ist dabei so geformt, dass ein Verrutschen des Dichtelements in axialer und transversaler Richtung verhindert wird. Die Verbindung von Zylinder und integrierter Verschlusskappe erfolgt bevorzugt über einen Bördelverschluss, wobei die integrierte Verschlusskappe in eine radiale Nut am distalen Ende des Zylinders (1) eingreift. Ein weiteres Beispiel für eine Verbindung ist der Schraubverschluss.

In einer weiteren nicht erfindungsgemäßen Ausführungsform besteht der Aufsatz aus einem einstückigen Verschlusselement (16) gekennzeichnet durch eine Bördelkappe mit einer distalen Öffnung zum Aufsetzen auf den Zylinder (1) und ein Dichtelement (15) zum Abdichten des distalen Endes des Verschlusselementes (16) und zum Abdichten der Verbindung zwischen Verschlusselement (16) und Zylinder (1). Das Verschlusselement (16) ist dabei so geformt, dass ein Verrutschen des Dichtelements in axialer und transversaler Richtung verhindert wird. Die Verbindung von Zylinder (1) und Verschlusselement (16) erfolgt bevorzugt über einen Bördelverschluss, wobei Verschlusselement (16) in eine radiale Nut am distalen Ende des Zylinders (1) eingreift. Ein weiteres Beispiel für eine Verbindung ist der Schraubverschluss.
In einer weiteren nicht erfindungsgemäßen Ausführungsform besteht der Aufsatz aus einem einstückigen Verschlusselement (16) mit distaler Öffnung, einem Dichtelement (15) zum Abdichten des distalen Endes des Verschlusselementes (16) und zum Abdichten der Verbindung zwischen Verschlusselement (16) und Zylinder (1). In dieser Ausführungsform besitzt der Zylinder (1) einen im wesentlichen zylindrischen Grundkörper und einem distalen Teilstück mit einem gegenüber dem Durchmesser des Grundkörpers verringerten Durchmesser, das mit einer distalen Öffnung geeignet zum Durchstechen mit einer Applikationsnadel und optional einem Nadelhalter versehen ist. Beispielsweise verjüngt sich der Grundkörper über eine Schulter zu dem Teilstück mit verringertem Durchmesser. Der Durchmesser der distalen Öffnung ist vorzugsweise 6-10 mm, besonders bevorzugt 6-9 mm, speziell bevorzugt 7-9 mm. Beispielsweise kann der Durchmesser der distalen Öffnung bei einem Außendurchmessers des Zylinders (1) von 9-15 mm 6-9, vorzugsweise 6-7 mm betragen. Die distale Öffnung gewährleistet ein leichtes Befüllen der Karpule mit der trockenen Wirkstoffzubereitung, insbesondere mit einem Pulver. Das Verschlusselement (16) ist dabei so geformt, dass ein Verrutschen des Dichtelements in axialer und transversaler Richtung verhindert wird. Die Verbindung von Zylinder (1) und Verschlusselement (16) erfolgt bevorzugt über einen Bördelverschluss, wobei Verschlusselement (16) über eine Wulst am distalen Ende des Teilstückes greift. Ein weiteres Beispiel für eine Verbindung von Zylinder (1) und Verschlusselement (16) ist der Schraubverschluss.

In einer weiteren bevorzugten Ausführungsform ist der Verschluss zweistückig ausgeführt.

Vorzugsweise enthält die zweistückige Ausführungsform ein Dichtelement zum Abdichten des distalen Ende des Verschlusses und ein weiteres zum Abdichten der Verbindung zwischen Verschluss und Zylinder. Das Dichtungselement zum Abdichten der Verbindung zwischen Verschluss und Zylinder ist bei zylindrischem Querschnitt von Zylinder und Verschluss beispielsweise ein Dichtring. Das Dichtungselement zum Abdichten des distalen Ende des Verschlusses ist bevorzugt eine Dichtscheibe.

Besonders bevorzugt besteht der Verschluss aus einem Kopf (2) und einer Verschlusskappe (5). Der Aufsatz besteht folglich aus einem Kopf (2) und einer Verschlusskappe (5) mit distaler Öffnung geeignet zum Durchstechen mit einer Applikationsnadel und optional einem Nadelhalter, einem Dichtelement zum Abdichten der Verbindung zwischen Kopf (2) und Verschlusskappe (5) und optional einem weiteren Dichtelement (7) zum Abdichten der Verbindung zwischen Kopf (2) und Zylinder (1). Die Verbindung von Zylinder und Kopf erfolgt vorzugsweise durch Eingriff des Kopfes (2) in den Zylinder (1) oder durch Eingriff des Zylinders (1) in den Kopf (2). Der Kopf (2) besitzt am proximalen Ende eine zum distalen Ende des Zylinders (1) kompatible Geometrie, beispielsweise bei einer zylinderischen Grundform von Zylinder (1) einen zum distalen Ende des Zylinders kompatiblen Durchmesser. Zylinder (1) ist bevorzugt von zylinderischer Grundform und besitzt besonders bevorzugt einen im wesentlichen über die gesamte Länge konstanten Durchmesser. Besonders bevorzugt sind Kopf und Zylinder mittels Klemmverschluss oder insbesondere bei zylinderischer Geometrie mittels Schraubverschluss miteinander verbunden.

In einer weiteren bevorzugten Ausführungsform ist der Verschluss dreistückig ausgeführt.

Vorzugsweise enthält die dreistückige Ausführungsform ein Dichtelement zum Abdichten des distalen Ende des Verschlusses und ein weiteres zum Abdichten der Verbindung zwischen Verschluss und Zylinder. Das Dichtungselement zum Abdichten der Verbindung zwischen Verschluss und Zylinder ist bei zylindrischem Querschnitt von Zylinder und Verschluss beispielsweise ein Dichtring. Das Dichtungselement zum Abdichten des distalen Ende des Verschlusses ist bevorzugt eine Dichtscheibe.

Bevorzugt besteht der Verschluss aus einem Kopf (2), einem Kopfhalter (19) und einer Verschlusskappe (5). Der Aufsatz besteht folglich aus einem Kopf (2), einem Kopfhalter (19) und einer Verschlusskappe (5) mit distaler Öffnung geeignet zum Durchstechen mit einer Applikationsnadel und optional einem Nadelhalter, einem Dichtelement zum Abdichten der Verbindung zwischen Kopf (2) und Verschlusskappe (5) und optional einem weiteren Dichtelement (7) zum Abdichten der Verbindung zwischen Kopf (2) und Zylinder (1). Die Verbindung von Zylinder und Verschluss erfolgt bevorzugt über einen Bördelverschluß, wobei der Kopfhalter (19) in eine radiale Nut am distalen Ende des Zylinders (1) eingreift. Ein weiteres Beispiel für eine Verbindung ist der Schraubverschluss. Vorzugsweise wird eine transversale Bewegung des Kopfes gegenüber dem Zylinder durch Eingriff des Kopfes (2) in den Zylinder (1) oder durch Eingriff des Zylinders (1) in den Kopf (2) verhindert.

Die Verschlusskappe (5) ist eine Vorrichtung, die das distale Ende des Kopfes (2) dicht verschließt. Die Kappe besteht aus Aluminium oder einem Kunststoff und gewährleistet dauerhafte Verbindung und Dichtkraft mit dem Kopf (2). Die Verbindung kann durch dem Fachmann bekannte Methoden, beispielsweise durch Crimpen, Bördeln, Aufprellen oder Verschrauben hergestellt werden.

In einer speziell bevorzugten Ausführungsform eines zweistückigen oder dreistückigen Verschlusses enthält entweder das proximale Ende des Kopfes (2) oder das distale Ende des Zylinders (1) eine axiale Nut (11) an der Innenseite des aufnehmenden Bauteils. Bei dem Zusammenfügen Zylinder (1) und Kopf (2), kann sich ein Druck in Kammer (17) aufbauen, der den Zwischenstopfen (6) und gegebenenfalls auch den Endstopfen (10) herausdrücken könnte. Der Druck kann durch die axiale Nut (11) weitgehend vermieden werden, da beim Zusammenfügen zunächst Luft entweichen kann. Die Nut ist so ausgeformt, dass sie axial eine Länge hat, die geringer ist als die Länge des Eingriffs des proximalen Endes des eingreifenden Bauteils in das entsprechende Ende des aufnehmenden Bauteils. Ferner ist die Nut so ausgeformt, dass sie die Wandstärke des aufnehmenden Bauteils weiter verringert. Greift beispielsweise wie in Figur 1a dargestellt das proximale Ende des Kopfes (2) in das distale Ende des Zylinders (1) ein, kann der Druck, der beim Zusammenfügen aufgebaut wird, zunächst durch die Nut (11) entweichen. Erst im letzten Schritt des Zusammenbaus kann der Druck nicht mehr über die Nut entweichen, ist aber so gering, dass er die Position des Zwischenstopfens (6) und des Endstopfens (10) nicht mehr verändern kann. In einer besonders bevorzugten Ausführungsform werden Zylinder (1) und Kopf (2), von denen einer eine axiale Nut enthält, mittels Klemmschluss miteinander verbunden.

Die axiale Nut (11) kann am proximalen Ende des Kopfes (2) oder am distalen Ende des Zylinders (1) sein; vorzugsweise ist die axiale Nut (11) am distalen Ende des Zylinders (1).

In einer bevorzugten Ausführungsform enthält die Zweikammerkarpule eine axiale Nut (11) im Zylinder (1) und einen Anschlag (13) am proximalen Ende des Zylinders (1).

In einer bevorzugten Ausführungsform eines zweistückigen oder dreistückigen Verschlusses sind Zylinder (1) und Kopf (2) aus Kunststoff. In einer besonders bevorzugten Ausführungsform sind beide Kunststoff-Bauteile über einen Schraubverschluss miteinander verbunden, wobei optional zur Abdichtung zwischen die Teilzylinder ein Dichtungselement eingefügt ist. Speziell bevorzugt enthält in dieser Ausführungsform das distale Ende des Zylinders (1) eine axiale Nut (11), über die beim Zusammenbau der beiden Bauteile der sich in der Kammer (17) aufgebaute Druck entweichen kann; alternativ können der Zwischenstopfen (6) und der Endstopfen (10) so im Zylinder (1) positioniert werden, dass beide beim Zusammenbau durch den aufgebauten Druck in die gewünschte Endlage verschoben werden. Vorzugsweise enthält Zylinder (1) am proximalen Ende einen Anschlag (13).

In einer weiteren bevorzugten Ausführungsform eines zweistückigen oder dreistückigen Verschlusses besteht der Zylinder (1) aus Glas und der Kopf (2) aus Kunststoff, die besonders bevorzugt über einen Klemmverschluss miteinander verbunden sind. Die Abdichtung der Teilzylinder kann über Verkleben mittels konventionellen pharmatauglichen Klebstoffs erfolgen, wobei optional zwischen Zylinder und Kopf ein Dichtungselement eingefügt ist. Speziell bevorzugt enthält in dieser Ausführungsform das distale Ende des Zylinders (1) eine axiale Nut (11), über die beim Zusammenbau der beiden Bauteile der sich in der Kammer (17) aufgebaute Druck entweichen kann; alternativ können der Zwischenstopfen (6) und der Endstopfen (10) so im Zylinder (1) positioniert werden, dass beide beim Zusammenbau durch den aufgebauten Druck in die gewünschte Endlage verschoben wird. Vorzugsweise enthält Zylinder (1) am proximalen Ende einen Anschlag (13).

In einer weiteren bevorzugten Ausführungsform eines zweistückigen oder dreistückigen Verschlusses bestehen beide Zylinder (1) und Kopf (2) aus Kunststoff und sind über einen Steckverschluss miteinander verbunden, wobei optional zur Abdichtung zwischen die Teilzylinder ein Dichtungselement eingefügt ist. Speziell bevorzugt enthält in dieser Ausführungsform das distale Ende des Zylinders (1) eine axiale Nut (11), über die beim Zusammenbau der beiden Bauteile der sich in der Kammer (17) aufgebaute Druck entweichen kann; alternativ können der Zwischenstopfen (6) und der Endstopfen (10) so im Zylinder (1) positioniert werden, dass beide beim Zusammenbau durch den aufgebauten Druck in die gewünschte Endlage verschoben wird. Vorzugsweise enthält Zylinder (1) am proximalen Ende einen Anschlag (13).

Kopfhalter (19) besteht beispielsweise aus Metall oder Kunststoff, bevorzugt aus Aluminium.

Generell ist eine Kombination aller der genannten allgemeinen und bevorzugten Merkmale der Ausführungsformen technisch möglich.

Besonders bevorzugt sind die folgenden Ausführungsvarianten:
1.) Der Verschluss ist zweistückig ausgeführt, das Fertigungsmaterial des Zylinders (1) und des Kopfes (2) ist Kunststoff und die Verbindung des Zylinders mit dem Kopf wird über einen Schraubverschluss herbeigeführt. Die Verbindung ist entweder selbstdichtend oder die Abdichtung der Verbindung wird über ein Dichtungselement (7) aus geeignetem Material gewährleistet.
2.) Der Verschluss ist zweistückig ausgeführt, das Fertigungsmaterial des Zylinders (1) ist Glas und das des Kopfes (2) ist Kunststoff; die Verbindung der beiden Bauteile (1) und (2) wird über einen Klemmverschluss herbeigeführt. Die Verbindung ist entweder selbstdichtend oder die Abdichtung der Verbindung wird mittels Verkleben mit einem konventionellen pharmatauglichen Klebstoffs gewährleistet.
3.) Der Verschluss ist zweistückig ausgeführt, das Fertigungsmaterial des Zylinders (1) und des Kopfes (2) ist Kunststoff und die Verbindung des Zylinders mit dem Kopf wird über einen Klemmverschluss herbeigeführt. Die Verbindung ist entweder selbstdichtend oder die Abdichtung der Verbindung wird mittels Ultraschallschweißen gewährleistet.
4.) Der Verschluss ist zweistückig ausgeführt, das Fertigungsmaterial des Zylinders (1) und des Kopfes (2) ist Glas und die Verbindung des Zylinders mit dem Kopf wird über einen Bördelverschluss herbeigeführt. Die Abdichtung der Verbindung wird vorzugsweise über ein Dichtungselement (7) aus geeignetem Material gewährleistet.
5.) Der nicht erfindungsgemäße Verschluss ist einstückig ausgeführt, das Fertigungsmaterial des Zylinders (1) ist Glas oder Kunststoff und der Verschluss ist eine integrierte Verschlusskappe (22) oder ein einstückiges Verschlusselement (16) aus Metall, bevorzugt auf der Innenseite mit einer Schutz-Lackierung versehenes Aluminium. Die Verbindung ist vorzugsweise zusätzlich über ein Dichtungselement gewährleistet sein. Das proximale Ende des einstückigen Verschlusselements (16) ist bevorzugt mit einer radialen Nut (20) am distalen Ende (8) des Zylinders gecrimpt. Sofern der Kopf als integrierte Verschlusskappe (22) ausgebildet ist, ist das proximale Ende des integrierten Aufsatzes (22) bevorzugt mit einer radialen Nut (20) am distalen Ende (8) des Zylinders gecrimpt.
6.) Der nicht erfindungsgemäße Verschluss ist einstückig ausgeführt, das Fertigungsmaterial des Zylinders (1) ist Glas oder Kunststoff und der Zylinder (1) besitzt einen im wesentlichen zylindrischen Grundkörper und ein distales Teilstück mit einem gegenüber dem Durchmesser des Grundkörpers verringerten Durchmesser, das mit einer distalen Öffnung geeignet zum Durchstechen mit einer Applikationsnadel und optional einem Nadelhalter versehen ist, wobei sich der Grundkörper über eine Schulter zu dem Teilstück mit verringertem Durchmesser verjüngt. Die Verbindung von Zylinder (1) und Verschlusselement (16) erfolgt über einen Bördelverschluss oder einen Schraubverschluss.

Allen Ausführungsformen ist gemein, dass die flüssige Komponente (14) und die feste Komponente (12) bevorzugt unter sterilen Bedingungen abgefüllt werden.

Die erfindungsgemäße Zweikammerkarpule hat den Vorteil, dass der Zylinder durch den gesamten Durchmesser der Teilzylinder befüllbar ist, und dass der Aufsatz nach Befüllen der Kammer (17) ohne Kontaminationsgefahr durch die flüssige Komponente (14) an den Kontaktstellen zwischen den Bauteilen aufgesetzt werden kann. Die Befüllung über große Öffnungen gewährleistet eine verringerte Wahrscheinlichkeit einer Kontamination des jeweils anderen Teilzylinders bzw. der Karpulenaußenseite. Da Pulver direkt befüllt werden können, ist kein Lyophilisieren notwendig. Stattdessen kann direkt ein Lyophilisat (das Produkt einer Lyophilisierung) oder eine andere feste Komponente, vorzugsweise in Pulverform, eingefüllt werden. Die schonende Pulverbefüllung gewährleistet ferner, dass keine Beeinflussung der kristallinen Struktur des Pulvers über Scherkräfte auftreten, wie sie beim Befüllen durch kleine Öffnungen auftreten können, so dass keine Beeinflussung der Bioverfügbarkeit der zu verabreichenden Medikamente zu erwarten ist. Ferner ist der Ort der Trennung der beiden Bauteile Zylinder und Aufsatz im Bereich der kopfseitigen Kammer (17), so dass bei Pulverbefüllung einfacher ein steriler Prozess gewährleistet werden kann. Durch das Setzen des mittleren Stopfens nach Befüllen der kolbenseitigen Kammer (18) ist eine Kontaminationsgefahr beider Komponenten (12) und (14) ausgeschlossen.

Die erfindungsgemäße Zweikammerkarpule ist in jedes Applikations-System, beispielsweise ein Pen-System einsetzbar, wobei das Applikations-System vorzugsweise eine Nadel zum Durchstoßen einer distalen Dichtscheibe enthält, sowie einen Antriebsmechanismus zum Bewegen des Endstopfens (10) in distale Richtung.

Vorzugsweise werden Zylinder (1) und Kopf (2) im Reinraum spritzgegossen, danach magaziniert in hermetischer Verpackung sterilisiert.

Die Verwendung von Kunststoff als Material für die Teilzylinder gewährleistet darüber hinaus die kostengünstige Herstellung der Teile, die Integration von Funktionsteilen an einen der Teilzylinder, die für das Betreiben eines Pen-Systemes erforderlich sind (Synergien), einen einfachen Prozess zur Herstellung von Partikelfreiheit, Sterilisation, Entpyrogenisieren, hohe Maßhaltigkeit und Recycelfähigkeit.

Die Erfindung betrifft ferner ein Applikationsgerät enthaltend eine Zweikammerkarpule wie oben beschrieben.

Ein weiterer Gegenstand der Erfindung ist ein Verfahren zum Herstellen und Befüllen einer Zweikammerkarpule, dadurch gekennzeichnet, dass
a) ein Zylinder enthaltend einen Bypass mit einem Mittelstopfen zwischen Bypass und proximalem Ende versehen wird;
b) die Kammer zwischen Mittelstopfen und proximalem Ende mit einer flüssigen Komponente befüllt wird;
c) die die flüssige Komponente enthaltende Kammer mit einem Endstopfen verschlossen wird;
d) eine flüssige oder bevorzugt feste Komponente in die Kammer zwischen Mittelstopfen und distalem Ende des Zylinders eingefüllt wird; und
e) das distale Ende des Zylinders mit einem Aufsatz verschlossen wird.

Die Komponenten bzw. Bauteile der Zweikammerkarpule sind wie oben in ihren allgemeinen und bevorzugten Ausführungsformen definiert.

In einer bevorzugten Ausführungsform wird der Mittelstopfen vor dem Einfügen des Endstopfens so positioniert, dass der aufgebaute Druck den Mittelstopfen in die gewünschte Endlage verschiebt. Der Mittelstopfen kann prinzipiell von beiden Enden des Zylinders eingebracht werden.

In einer weiteren bevorzugten Ausführungsform werden Zylinder und Aufsatz unter Vakuumbedingungen zusammengesetzt. Durch diese Maßnahme wird ein Verschieben der Stopfen weitgehend vermieden.

Dem Fachmann stehen eine Reihe bekannter Möglichkeiten zum Zusammenfügen von Zylinder und Aufsatz zur Verfügung. Es können stoffschlüssige, kraftschlüssige oder formschlüssige Verbindungstechniken Verwendung finden, wobei ihre Eignung von den jeweils verwendeten Werkstoffen für die Spritzenteilzylinder abhängt. Beispielsweise sind geeignet: Verkleben, Verschweißen, Klemmverschluss, Schraubverschluss, Steckverschluss, Bajonettverschluss, Schnappverschluss, Prellverschluss und Bördelverschluss (= Crimpverschluss). Sind beide Teilzylinder aus Kunststoff, kommt insbesondere eine Schweißverbindung in Frage; aber auch eine Verklebung ist möglich, eine Verbindungstechnik, die auch dann angewendet werden kann, wenn beide Teilzylinder aus Glas bestehen. In einer weiteren Ausgestaltung können die beiden Zylinderteile, gleichgültig ob sie aus Glas oder Kunststoff hergestellt sind, mittels einer dichtenden Schnappverbindung miteinander verbunden sein oder ein Dichtungselement enthalten. Sofern der Verschluss dreistückig aufgebaut ist und als zusätzliches Bauteil neben Kopf (2) und Verschlusskappe (5) einen Kopfhalter (19) enthält, kann das proximale Ende des Kopfhalters (19) mit einer radialen Nut (20) am distalen Ende (8) des Zylinders gecrimpt sein. Sofern der Aufsatz aus einem einstückigen Verschlusselement (16) und einer Dichtscheibe (15) gebildet wird, kann das proximale Ende des Verschlusselements (16) mit einer radialen Nut (20) am distalen Ende (8) des Zylinders gecrimpt sein. Sofern der Verschluss als integrierte Verschlusskappe (22) gebildet wird, kann das proximale Ende des integrierten Verschlusskappe (22) mit einer radialen Nut (20) am distalen Ende (8) des Zylinders gecrimpt kann.

Die Figuren 1 a und 1 b zeigen den prinzipiellen Aufbau der Erfindung als Explosionszeichnung (Figur 1 a) und im montierten Zustand (Figur 1 b). Figur 1 c zeigt den Aufbau einer nicht erfindungsgemäßen Verschlusskappe (5). Dieser Aufbau entspricht dem Aufbau eines einstückigen Verschlusselementes.

Die Figuren 2a bis 2c zeigen die Verbindung des Kopfes (2) mit dem Zylinder (1) mittels Bördelverschluss über einen Kopfhalter (19). Figur 2b zeigt einen vormontierten Kopf mit Verschlusskappe (21), oberer Teil.

Figur 3a zeigt eine nicht erfindungsgemäße integrierte Verschlusskappe (22), die mittels Bördelverschluss am Zylinder befestigt werden kann (Figur 3b). Figur 3b zeigt die integrierte Verschlusskappe (22) im mit dem Zylinder verbundenen Zustand (gecrimpt).

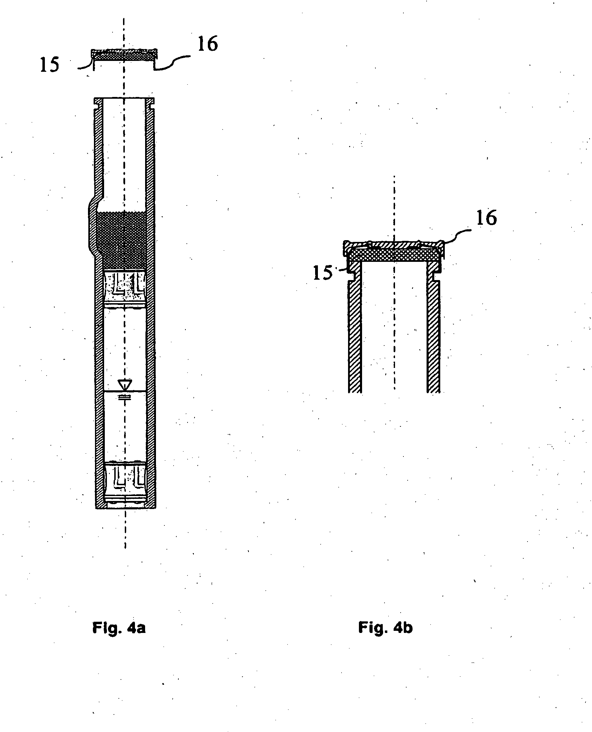

Figur 4a zeigt als einstückigen Aufsatz ein nicht erfindungsgemäßes Verschlusselement (16) und eine Dichtscheibe (15). Figur 4b zeigt das nicht erfindungsgemäße einstückige Verschlusselement (16) und die Dichtscheibe (15) im mit dem Zylinder verbundenen Zustand (gecrimpt).

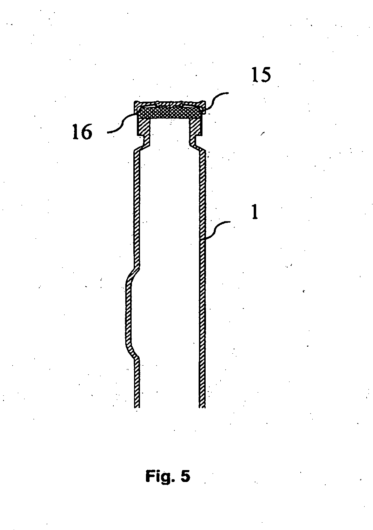

Figur 5 zeigt den oberen Teil einer Zweikammerkarpule mit einer weiteren Ausführungsform eines nicht erfindungsgemäßen einstückigen Aufsatzes als Verschlusselement (16), einen Zylinder (1) und eine Dichtscheibe (15), wobei der Zylinder (1) einen im wesentlichen zylindrischen Grundkörper und ein distales Teilstück mit einem gegenüber dem Durchmesser des Grundkörpers verringerten Durchmesser besitzt, das mit einer distalen Öffnung geeignet zum Durchstechen mit einer Applikationsnadel und optional einem Nadelhalter versehen ist. Das Verschlusselement (16) ist über eine Wulst des Zylinders gebördelt.

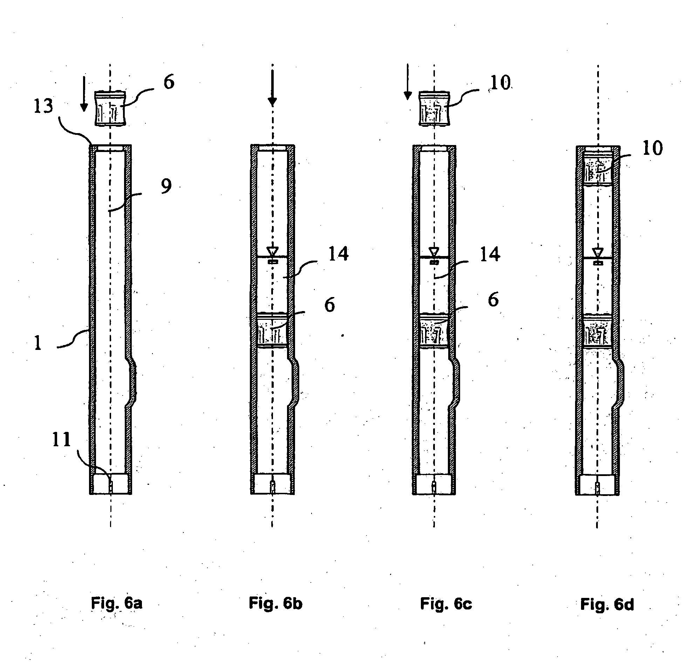

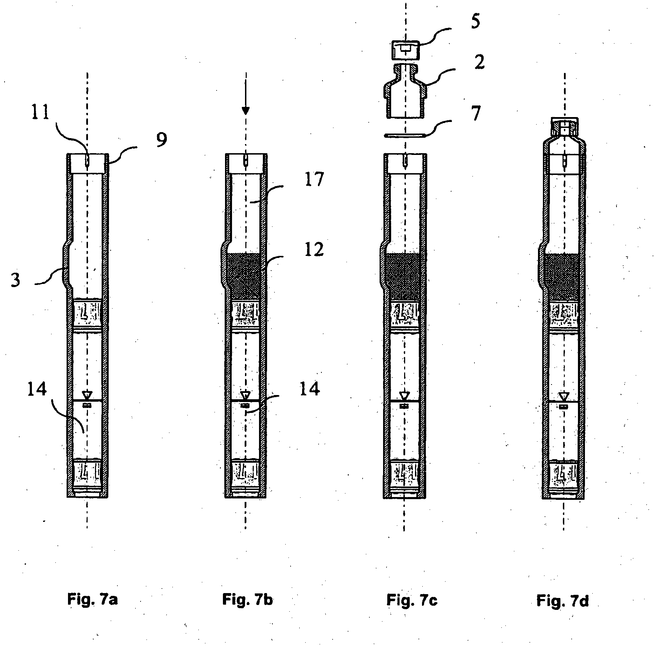

Die Figuren 6a-7d zeigen ein Verfahren zur Befüllung der erfindungsgemäßen Zweikammerkarpule. Figur 6a zeigt das Einfügen des Mittelstopfens (6) in den Zylinders (1). Figur 6b zeigt das Befüllen der kolbenseitigen Kammer (18) mit einem flüssigen Trägermedium (14). Figur 6c zeigt das Verschließen der Kammer (18) mit dem Endstopfens (10). Figur 6d zeigt Zylinder (1) mit Mittelstopfen (6), Endstopfen (10) und befüllter kolbenseitiger Kammer (18). Figur 7a zeigt den zur weiteren Bearbeitung gedrehten Zylinder (1) mit Mittelstopfen (6), Endstopfen (10) und befüllter kolbenseitiger Kammer (18), bei dem das kopfseitige Ende nach oben zeigt. Figur 7b zeigt das Einbringen der trockenen Wirkstoffzubereitung (12). Figur 7c zeigt das Plazieren von Kopf (2), Verschlusskappe (5) und Dichtungselement (7) am distalen Ende (8) des Zylinders zur gebrauchsfähigen, verschlossenen Zweikammerkarpule.

Der Montage- und Befüllweg der Zweikammerkarpule ist im Folgenden beispielhaft beschrieben (Figuren 6-7):
1. Einsetzen des Mittelstopfens (6) in den Zylinder (1). Der Mittelstopfens (6) kann von beiden Enden des Zylinders (1) eingebracht werden, wobei das bevorzugte Ende das proximales Ende darstellt (Figur 6a).
2. Wenn der Mittelstopfens (6) in seine Position gebracht ist, wird der Zylinder (1) mit flüssigem Trägermedium (14) gefüllt (Figur 6b). Die Größe der Kammer (18) wird über das Volumen des einzufüllenden Trägermediums (14) bedingt.
3. Wenn das flüssigem Trägermedium (14) eingefüllt ist, wird das proximale Ende (9) des Zylinders mit einem Endstopfen (10) verschlossen (Figur 6c).
4. Der mit flüssigem Trägermedium (14) gefüllte und mit Mittel- und Endstopfen versehener Zylinder (1) wird um 180° gedreht, so dass das distale Ende nach oben zeigt (Figur 7a).
5. Der Zylinder (1) wird mit der trockenen Wirkstoffzubereitung (12) gefüllt (Figur 7b).
6. Am distalen Ende (8) des Zylinders (1) wird der Kopf (2) angebracht und verbunden (Figur 7c). Sofern ein Dichtungselement (7) verwendet wird, kann dieses platziert werden. Auf dem Kopf (2) kann bereits eine Verschlusskappe (5) vormontiert sein.
7. Sofern Kopf (2) und Verschlusskappe (5) nicht bereits vormontiert in Schritt 6. eingesetzt werden, wird anschließend auf dem Kopfende die Verschlusskappe (5) aufgesetzt und mit dem Kopf (2) verbunden.

### Glossar:

- (1): Zylinder
- (2): Kopf
- (3): Bypass
- (4): Öffnung
- (5): Verschlusskappe
- (6): Mittelstopfen
- (7): Dichtungselement
- (8): distales Ende
- (9): proximales Ende
- (10): Endstopfen
- (11): axiale Nut
- (12): trockene Wirkstoffzubereitung
- (13): Anschlag
- (14): flüssiges Trägermedium
- (15): Dichtscheibe
- (16): Verschlusselement
- (17): kopfseitige Kammer
- (18): kolbenseitige Kammer
- (19): Kopfhalter
- (20): radiale Nut
- (21): vormontierter Kopf mit Verschlusskappe
- (22): integrierte Verschlusskappe mit Bördelvorrichtung

## Patentansprüche

1. Zweikammerkarpule bestehend aus
a) einem Zylinder enthaltend
einen Endstopfen, der am proximalen Ende des Zylinders positioniert ist, optional einen Anschlag (13) am proximalen Ende (9),
einen Zwischenstopfen (6),
einen Bypass (3),
ein distales Ende (8) zum Verbinden mit einem Aufsatz, und
b) dem Aufsatz bestehend aus
einem Verschluss mit einer distalen Öffnung geeignet zum Durchstechen mit einer Applikationsnadel und optional einem Nadelhalter,
einem oder mehreren Dichtelemente zum Abdichten des distalen Endes des Verschlusses und/oder zum Abdichten der Verbindung zwischen Verschluss und Zylinder,
**dadurch gekennzeichnet dass,**
der Verschluss zweistückig oder dreistückig ausgeführt ist und
ein proximales Ende des Aufsatzes das Ende eines Kopfes ist, das im zusammengebauten Zustand mit dem distalen Ende des Zylinders verbunden ist, wobei eine axiale Nut (11) am proximalen Ende des Kopfes oder am distalen Ende des Zylinders an der Innenseite des aufnehmenden Bauteils vorgesehen ist.

2. Zweikammerkarpule gemäß Anspruch 1,
wobei der Aufsatz aus dem Kopf (2) und einer Verschlusskappe (5) mit distaler Öffnung geeignet zum Durchstechen mit einer Applikationsnadel und optional einem Nadelhalter, einem Dichtelement zum Abdichten der Verbindung zwischen Kopf (2) und Verschlusskappe(5) und optional einem weiteren Dichtelement (7) zum Abdichten der Verbindung zwischen Kopf (2) und Zylinder (1) besteht.

3. Zweikammerkarpule gemäß Anspruch 1,
wobei der Aufsatz aus dem Kopf (2), einem Kopfhalter (19) und einer Verschlusskappe (5) mit distaler Öffnung geeignet zum Durchstechen mit einer Applikationsnadel und optional einem Nadelhalter, einem Dichtelement zum Abdichten der Verbindung zwischen Kopf (2) und Verschlusskappe (5) und optional einem weiteren Dichtelement (7) zum Abdichten der Verbindung zwischen Kopf (2) und Zylinder (1) besteht.

4. Zweikammerkarpule gemäß einem der Ansprüche 1 bis 3,
wobei das proximale Ende des Zylinders (1) einen Anschlag (13) aufweist.

5. Zweikainmerkarpule gemäß einem der Ansprüche 1 bis 4,
wobei die Zweikammerkarpule ein zu verabreichendes Medikament aufweist, und wobei das zu verabreichende Medikament ein Peptid zur Behandlung von Diabetes mellitus oder Komplikationen von Diabetes mellitus ist.

6. Zweikammerkarpule gemäß Anspruch 5,
wobei das Peptid Exendin-4(1-39) oder ein Peptid ausgewählt der Gruppe
H-(Lys)₄-des Pro³⁶, des Pro³⁷ Exendin-4(1-39)-NH₂,
H-(Lys)₅-des Pro³⁶, des Pro³⁷ Exendin-4(1-39)-NH₂,
des Pro³⁶ [Asp²⁸] Exendin-4(1-39),
des Pro³⁶ [IsoAsp²⁸] Exendin-4(1-39),
des Pro³⁶ [Met(O)¹⁴, Asp²⁸] Exendin-4(1-39),
des Pro³⁶ [Met(O)¹⁴, IsoAsp²⁸] Exendin-4(1-39),
des Pro³⁶ [Trp(O₂)²⁵, Asp²⁸] Exendin-4(1-39),
des Pro³⁶ [Trp(O₂)²⁵, IsoAsp²⁸] Exendin-4(1-39),
des Pro³⁶ [Met(O)¹⁴ Trp(O₂)²⁵, Asp²⁸] Exendin-4 (1-39),
des Pro³⁶ [Met(O)¹⁴ Trp(O₂)²⁵, IsoAsp²⁸] Exendin-4(1-39),
H-(Lys)₆-des Pro³⁶ [Asp²⁸] Exendin-4(1-39)-Lys₆-NH₂,
desAsp²⁸ Pro³⁶, Pro³⁷, Pro³⁸Exendin-4(1-39)-NH₂,
H-(Lys)₆-des Pro³⁶, Pro³⁸ [Asp²⁸] Exendin-4(1-39)-NH₂,
H-Asn-(Glu)₅des Pro³⁶, Pro³⁷, Pro³⁸ [Asp²⁸] Exendin-4(1-39)-NH₂,
des Pro³⁶, Pro³⁷, Pro³⁸ [Asp²⁸] Exendin-4(1-39)-(Lys)₆-NH₂,
H-(Lys)₆-des Pro³⁶, Pro³⁷, Pro³⁸ [Asp²⁸] Exendin-4 (1-39)-(Lys)₆-NH₂,
H-Asn-(Glu)₅-des Pro³⁶, Pro³⁷, Pro³⁸ [Asp²⁸] Exendin-4(1-39)-(Lys)₆-NH₂,
H-(Lys)₆-des Pro³⁶ [Trp(O₂)²⁵, Asp²⁸] Exendin-4(1-39)-Lys₆-NH₂,
H-des Asp²⁸ Pro³⁶, Pro³⁷, Pro³⁸ [Trp(O₂)²⁵] Exendin-4(1-39)-NH₂,
H-(Lys)₆-des Pro³⁶, Pro³⁷, Pro³⁸ [Trp(O₂)²⁵, Asp²⁸] Exendin-4(1-39)-NH₂,
H-Asn-(Glu)₅-des Pro³⁶, Pro³⁷, Pro³⁸ [trp(O₂)²⁵,
Asp²⁸] Exendin-4(1-39)-NH₂,
des Pro³⁶, Pro³⁷, Pro³⁸ [Trp(O₂)²⁵, Asp²⁸] Exendin-4(1-39)-(Lys)₆-NH₂,
H-(Lys)₆-des Pro³⁶, Pro³⁷, Pro³⁸ [trp(O₂)²⁵, Asp²⁸] Exendin-4(1-39)-(Lys)₆-NH2_{,}
H-Asn-(Glu)₅-des Pro³⁶, Pro³⁷, Pro³⁸ [Trp(O₂)²⁵,
Asp²⁸] Exendin-4(1-39)-(Lys)₆-NH_{2,}
H-(Lys)₆-des Pro³⁶ [Met(O)¹⁴, Asp²⁸] Exendin-4(1-39)-Lys₆-NH₂,
des Met(O)¹⁴ Asp²⁸ Pro³⁶, Pro³⁷, Pro³⁸ Exendin-4 (1-39)-NH₂,
H-(Lys)₆-desPro³⁶, Pro³⁷, Pro³⁸ [Met(O)¹⁴, Asp²⁸] Exendin-4(1-39)-NH_{2,}
H-Asn-(Glu)₅-des Pro³⁶, Pro³⁷, Pro³⁸ [Met(O)¹⁴,
Asp²⁸] Exendin-4(1-39)-NH₂,
des Pro³⁶, Pro³⁷, Pro³⁸ [Met(O)¹⁴ Asp²⁸] Exendin-4(1-39)-(Lys)₆-NH₂,
H-(Lys)₆-des Pro³⁶, Pro³⁷, Pro³⁸ [Met(O)¹⁴, Asp²⁸] Exendin-4(1-39)-(Lys)₆-NH₂,
H-Asn-(Glu)₅ des Pro³⁶, Pro³⁷, Pro³⁸ [Met(O)¹⁴,
Asp²⁸] Exendin-4(1-39)-(Lys)₆-NH₂,
H-Lys₆-des Pro³⁶ [Met(O)¹⁴, Trp(O₂)²⁵, Asp²⁸] Exendin-4(1-39)-Lys₆-NH₂,
H-des Asp²⁸ Pro³⁶, Pro³⁷, Pro³⁸ [Met(O)¹⁴, Trp (O₂)²⁵] Exendin-4(1-39)-NH₂,
H-(Lys)₆-des Pro³⁶, Pro³⁷, Pro³⁸ [Met(O)¹⁴, Asp²⁸] Exendin-4(1-39)-NH₂,
H-Asn-(Glu)₅-des Pro³⁶, Pro³⁷, Pro³⁸ [Met(O)¹⁴, Trp (O₂)²⁵, Asp²⁸] Exendin-4(1-39)-NH₂,
des Pro³⁶, Pro³⁷, Pro³⁸ [Met(O)¹⁴, Trp(O₂)²⁵, Asp²⁸] Exendin-4(1-39)-(Lys)₆-NH₂,
H-(Lys)₆-des Pro³⁶, Pro³⁷, Pro³⁸ [Met(O)¹⁴ Trp (O₂)²⁵, Asp²⁸] Exendin-4(S1-39)-(Lys)₆-NH₂,
H-Asn-(Glu)₅-des Pro³⁶, Pro³⁷, Pro³⁸ [Met(O)¹⁴ Trp (O₂)²⁵, Asp²⁸] Exendin-4(1-39)-(Lys)₆-NH₂: oder
des Pro³⁶ [Asp²⁸] Exendin-4(1-39),
des Pro³⁶ [IsoAsp²⁸] Exendin-4(1-39),
des Pro³⁶ [Met(O)¹⁴, Asp²⁸] Exendin-4(1-39),
des Pro³⁶ [Met(O)¹⁴, IsoAsp²⁸] Exendin-4(1-39),
des Pro³⁶ [Trp(O₂)²⁵, Asp²⁸] Exendin-4(1-39),
des Pro³⁶ [Trp(O₂)²⁵, IsoAsp²⁸] Exendin-4(1-39),
des Pro³⁶ [Met(O)¹⁴ Trp(O₂)², Asp²⁸] Exendin-4 (1-39),
des Pro³⁶ [Met(O)¹⁴ Trp(O₂)²⁵, IsoAsp²⁸] Exendin-4(1-39),
wobei die Gruppe -Lys₆-NH₂ mit dem C-Terminus des Exendin-4-Derivats verknüpft ist; oder ein pharmazeutisch akzeptables Salz oder Solvat davon ist.

7. Applikationsgerät enthaltend eine Zweikammerkarpule gemäß einem der Ansprüche 1 bis 6.

8. Verfahren zum Herstellen und Befüllen einer Zweikammerkarpule gemäß einem der Ansprüche 1 bis 6, **dadurch gekennzeichnet, dass**
a) ein Zylinder enthaltend einen Bypass mit einem Mittelstopfen zwischen Bypass und proximalem Ende versehen wird;
b) die Kammer zwischen Mittelstopfen und proximalem Ende mit einer flüssigen Komponente befüllt wird;
c) die die flüssige Komponente enthaltende Kammer mit einem Endstopfen verschlossen wird;
d) eine flüssige oder bevorzugt feste Komponente in die Kammer zwischen Mittelstopfen und distalem Ende des Zylinders eingefüllt wird; und
e) das distale Ende des Zylinders mit einem Aufsatz verschlossen wird.

## Claims

1. A two-chamber carpule comprising
a) a cylinder containing
an end plug, which is positioned at the proximal end of the cylinder,
optionally a stop (13) at the proximal end (9),
an intermediate plug (6),
a bypass (3),
a distal end (8) for connecting to an attachment, and
b) the attachment comprising
a closure with a distal opening suitable for piercing with an application needle and optionally a needle holder,
one or more sealing elements for sealing the distal end of the closure and for sealing the connection between the closure and the cylinder,
wherein
the closure is of a two-piece or three-piece configuration and
a proximal end of the attachment is the end of a head which, in the assembled state, is connected to the distal end of the cylinder, an axial groove (11) at the proximal end of the head or at the distal end of the cylinder being provided on the inner side of the receiving component.

2. The two-chamber carpule as claimed in claim 1,
the attachment comprising the head (2) and a closure cap (5) with a distal opening suitable for piercing with an application needle and optionally a needle holder, a sealing element for sealing the connection between the head (2) and the closure cap (5) and optionally a further sealing element (7) for sealing the connection between the head (2) and the cylinder (1).

3. The two-chamber carpule as claimed in claim 1,
the attachment comprising the head (2), a head holder (19) and a closure cap (5) with a distal opening suitable for piercing with an application needle and optionally a needle holder, a sealing element for sealing the connection between the head (2) and the closure cap (5) and optionally a further sealing element (7) for sealing the connection between the head (2) and the cylinder (1).

4. The two-chamber carpule as claimed in one of claims 1 to 3,
the proximal end of the cylinder (1) having a stop (13).

5. The two-chamber carpule as claimed in one of claims 1 to 4,
the two-chamber carpule having a medicament to be administered, and the medicament to be administered being a peptide for the treatment of Diabetes mellitus or complications of Diabetes mellitus.

6. The two-chamber carpule as claimed in claim 5,
the peptide being exendin-4(1-39) or a peptide selected from the group comprising
H-(Lys)₄-des Pro³⁶, des Pro³⁷ exendin-4(1-39)-NH₂,
H-(Lys)₅-des Pro³⁶, des Pro³⁷ exendin-4(1-39)-NH₂,
des Pro³⁶ [Asp²⁸] exendin-4(1-39),
des Pro³⁶ [IsoAsp²⁸] exendin-4(1-39),
des Pro³⁶ [Met(O)¹⁴, Asp²⁸] exendin-4(1-39),
des Pro³⁶ [Met(O)¹⁴, IsoAsp²⁸] exendin-4(1-39),
des Pro³⁶ [Trp(O₂)²⁵, Asp²⁸] exendin-4(1-39),
des Pro³⁶ [Trp(O₂)²⁵, IsoAsp²⁸] exendin-4(1-39),
des Pro³⁶ [Met(O)¹⁴ Trp(O₂)²⁵, Asp²⁸] exendin-4(1-39),
des Pro³⁶ [Met(O)¹⁴ Trp(O₂)²⁵, IsoAsp²⁸ exendin-4(1-39),
H-(Lys)₆-des Pro³⁶ [Asp²⁸] exendin-4(1-39)-Lys₆-NH₂,
des Asp²⁸ Pro³⁶, Pro³⁷, Pro38exendin-4(1-39)-NH₂,
H-(Lys)₆-des Pro³⁶, Pro³⁸ [Asp²⁸] exendin-4(1-39)-NH₂,
H-Asn-(Glu)₅des Pro³⁶, Pro³⁷, Pro³⁸ [Asp²⁸] exendin-4 (1-39)-NH₂,
des Pro³⁶, Pro³⁷, Pro³⁸ [Asp²⁸] exendin-4(1-39)-(Lys)₆-NH_{2,}
H-(Lys)₆-des Pro³⁶, Pro³⁷, Pro³⁸ [Asp²⁸] exendin-4(1-39) - (Lys) ₆-NH₂,
H-Asn-(Glu)₅-des Pro³⁶, Pro³⁷, Pro³⁸ [Asp²⁸] exendin-4 (1-39) - (Lys) ₆-NH₂,
H-(Lys)₆-des Pro³⁶ [Trp(O₂)²⁵, Asp²⁸] exendin-4(1-39)-Lys₆-NH₂,
H-des Asp²⁸ Pro³⁶, Pro³⁷, Pro³⁸ [Trp (O₂)²⁵] exendin-4 (1-39)-NH₂,
H-(Lys)₆-des Pro³⁶, Pro³⁷, Pro³⁸ [Trp(O₂)²⁵, Asp²⁸] exendin-4(1-39)-NH_{2,}
H-Asn-(Glu)₅-des Pro³⁶, Pro³⁷, Pro³⁸ [Trp(O₂)²⁵, Asp²⁸] exendin-4(1-39)-NH₂,
des Pro³⁶, Pro³⁷, Pro³⁸ [Trp(O₂)²⁵, Asp²⁸ exendin-4(1-39) -(Lys)₆-NH₂,
H-(Lys)₆-des Pro³⁶, Pro³⁷, Pro³⁸ [Trp(O₂)²⁵, Asp²⁸] exendin-4(1-39)-(Lys)₆,-NH₂,
H-Asn-(Glu)₅-des Pro³⁶, Pro³⁷, Pro³⁸ [Trp(O₂)²⁵, Asp²⁸] exendin-4(1-39)-(Lys)₆,-NH₂,
H-(Lys)₆-des Pro³⁶ [Met(O)¹⁴, Asp²⁸] exendin-4(1-39)-Lys₆-NH₂,
des Met(O)¹⁴ Asp²⁸ Pro³⁶, Pro³⁷, Pro³⁸ exendin-4(1-39) NH₂,
H-(Lys)₆-desPro³⁶, Pro³⁷, Pro³⁸ [Met(O)¹⁴, Asp²⁸] exendin-4(1-39)-NH₂,
H-Asn-(Glu)₅-des Pro³⁶, Pro³⁷, Pro³⁸ [Met(O)¹⁴, Asp²⁸] exendin-4(1-39)-NH₂,
des Pro³⁶, Pro³⁷, Pro³⁸ [Met(O)¹⁴, Asp²⁸] exendin-4(1-39) - (Lys) ₆-NH₂,
H-(Lys)₆-des Pro³⁶, Pro³⁷, Pro³⁸ [Met(O)¹⁴, Asp²⁸] exendin-4(1-39)-(Lys)₆-NH_{2,}
H-Asn-(Glu)₅ des Pro³⁶, Pro³⁷, Pro³⁸ [Met(O)¹⁴, Asp²⁸] exendin-4(1-39)-(Lys)₆-NH₂,
H-Lys₆-des Pro³⁶ [Met(O)¹⁴, Trp(O₂)²⁵, Asp²⁸] exendin-4 (1-39)-Lys₆-NH₂,
H-des Asp²⁸ Pro³⁶, Pro³⁷, Pro³⁸ [Met(O)¹⁴, Trp(O₂)²⁵] exendin-4(1-39)-NH₂,
H-(Lys)₆-des Pro³⁶, Pro³⁷, Pro³⁸ [Met(O)¹⁴, Asp²⁸] exendin-4(1-39)-NH₂,
H-Asn-(Glu)₅-des Pro³⁶, Pro³⁷, Pro³⁸ [Met(O)¹⁴,
Trp(O)²⁵, Asp²⁸] exendin-4(1-39)-NH₂,
des Pro³⁶, Pro³⁷, Pro³⁸ [Met(O)¹⁴, Trp(O)²⁵, Asp²⁸] exendin-4(1-39)-(Lys)₆,-NH₂,
H-(Lys)₆-des Pro³⁶, Pro³⁷, Pro³⁸ [Met(O)¹⁴, Trp(O)²⁵,
Asp²⁸] exendin-4(S1-39)-(Lys) ₆-NH₂,
H-Asn-(Glu)₅-des Pro³⁶, Pro³⁷, Pro³⁸ [Met(O)¹⁴,
Trp(O₂)²⁵, Asp²⁸] exendin-4(1-39)-(Lys)₆-NH₂; or
des Pro³⁶ [Asp²⁸] exendin-4(1-39),
des Pro³⁶ [IsoAsp²⁸] exendin-4(1-39),
des Pro³⁶ [Met(O)¹⁴, Asp²⁸] exendin-4(1-39),
des Pro³⁶ [Met(O)¹⁴, IsoAsp²⁸] exendin-4(1-39),
des Pro³⁶ [Trp(O₂)²⁵, Asp²⁸] exendin-4(1-39),
des Pro³⁶ [Trp(O₂)²⁵, IsoAsp²⁸] exendin-4(1-39),
des Pro³⁶ [Met(O)¹⁴ Trp(O₂)²⁵, Asp²⁸] exendin-4(1-39),
des Pro³⁶ [Met(O)¹⁴ Trp(O₂)²⁵, IsoAsp²⁸] exendin-4(1-39),
the group -Lys₆-NH₂ being linked with the C-terminus of the exendin-4 derivative;
or a pharmaceutically acceptable salt or solvate thereof.

7. An applicator including a two-chamber carpule as claimed in one of claims 1 to 6.

8. A method for producing and filling a two-chamber carpule as claimed in one of claims 1 to 6, wherein
a) a cylinder containing a bypass is provided with a middle plug between the bypass and the proximal end;
b) the chamber between the middle plug and the proximal end is filled with a liquid component;
c) the chamber containing the liquid component is closed with an end plug;
d) a liquid component, or with preference solid component, is filled into the chamber between the middle plug and the distal end of the cylinder; and
e) the distal end of the cylinder is closed with an attachment.

## Revendications

1. Ampoule à deux chambres, constituée
a) d'un cylindre contenant
un bouchon d'extrémité qui est positionné sur l'extrémité proximale du cylindre, en option, une butée (13) sur l'extrémité proximale (9),
un bouchon intermédiaire (6),
une dérivation (3),
une extrémité distale (8) destinée à être assemblée avec une garniture et
b) de la garniture constituée
d'une fermeture avec un orifice distal adapté pour être percé à l'aide d'une aiguille d'application et, en option, d'un porte-aiguille,
d'un ou de plusieurs éléments d'étanchéité pour assurer l'étanchéité de l'extrémité distale de la fermeture et/ou pour assurer l'étanchéité de la liaison entre la fermeture et le cylindre, **caractérisée en ce**
**que** la fermeture est conçue en deux pièces ou en trois pièces et
**qu'**une extrémité proximale de la garniture est l'extrémité d'une tête qui, à l'état assemblé, est reliée à l'extrémité distale du cylindre, une rainure axiale (11) étant prévue sur l'extrémité proximale de la tête ou sur l'extrémité distale du cylindre, sur la face intérieure de l'élément de réception.

2. Ampoule à deux chambres selon la revendication 1, la garniture étant constituée de la tête (2) et d'un capuchon de fermeture (5) avec un orifice distal adapté pour être percé à l'aide d'une aiguille d'application et, en option, d'un porte-aiguille, d'un élément d'étanchéité pour assurer l'étanchéité de la liaison entre la tête (2) et le capuchon de fermeture (5) et, en option, d'un élément d'étanchéité (7) supplémentaire pour assurer l'étanchéité de la liaison entre la tête (2) et le cylindre (1).

3. Ampoule à deux chambres selon la revendication 1, la garniture étant constituée de la tête (2), d'un support de tête (19) et d'un capuchon de fermeture (5) avec un orifice distal adapté pour être percé à l'aide d'une aiguille d'application et, en option, d'un porte-aiguille, d'un élément d'étanchéité pour assurer l'étanchéité de la liaison entre la tête (2) et le capuchon de fermeture (5) et, en option, d'un élément d'étanchéité (7) supplémentaire pour assurer l'étanchéité de la liaison entre la tête (2) et le cylindre (1).

4. Ampoule à deux chambres selon l'une quelconque des revendications 1 à 3,
l'extrémité proximale du cylindre (1) comportant une butée (13).

5. Ampoule à deux chambres selon l'une quelconque des revendications 1 à 4,
l'ampoule à deux chambres comportant un médicament à administrer et le médicament à administrer étant un peptide pour le traitement du diabète mellitus ou de complications du diabète mellitus.

6. Ampoule à deux chambres selon la revendication 5, le peptide étant de l'Exendine-4(1-39) ou un peptide choisi dans le groupe
H-(Lys)₄-du Pro³⁶, du Pro³⁷ Exendine-4(1-39)-NH₂,
H-(Lys)₅-du Pro³⁶, du Pro³⁷ Exendine-4(1-39)-NH₂,
du Pro³⁶ [Asp²⁸] Exendine-4(1-39),
du Pro³⁶ [IsoAsp²⁸] Exendine-4(1-39),
du Pro³⁶ [Met(O)¹⁴, Asp²⁸] Exendine-4(1-39),
du Pro³⁶ [Met(O)¹⁴, IsoAsp²⁸] Exendine-4 (1-39),
du Pro³⁶ [Trp(O₂)²⁵, Asp²⁸] Exendine-4(1-39),
du Pro³⁶ [Trp(O₂)²⁵, IsoAsp²⁸] Exendine-4(1-39),
du Pro³⁶ [Met(O)¹⁴ Trp(O)²⁵, Asp²⁸ Exendine-4(1-39),
du Pro³⁶ [Met(O)¹⁴ Trp(O)²⁵, IsoAsp²⁸] Exendine-4(1-39), H-(Lys)₆- du Pro³⁶ [Asp²⁸] Exendine-4(1-39)-Lys₆-NH₂,
de l'Asp²⁸ Pro³⁶, Pro³⁷, Pro³⁸ Exendine-4(1-39)-NH₂, H-(Lys)6- du Pro³⁶, Pro³⁸ [Asp²⁸] Exendine-4(1-39)-NH₂,
H-Asn-(Glu)₅ du Pro³⁶, Pro³⁷, Pro³⁸ [Asp²⁸] Exendine-4 (1-39)-NH₂,
du Pro³⁶, Pro³⁷, Pro³⁸ [Asp²⁸] Exendine-4(1-39)-(Lys) ₆-NH₂,
H-(Lys)₆- du Pro³⁶, Pro³⁷ Pro³⁸ [Asp²⁸] Exendine-4(1-39) - (Lys) ₆-NH₂,
H-Asn-(Glu)₅- du Pro³⁶, Pro³⁷, Pro³⁸ [Asp²⁸] Exendine-4(1-39)-(Lys)₆-NH₂,
H-(Lys)₆- du Pro³⁶ [Trp(O₂)²⁵, Asp²⁸] Exendine-4 (1-39) -Lys₆-NH₂,
H- de l'Asp²⁸ Pro³⁶, Pro³⁷, Pro³⁸ [Trp(O₂)²⁵] Exendine-4(1-39)-NH₂,
H-(Lys)₆- du Pro³⁶, Pro³⁷, Pro³⁸ [Trp(O₂)²⁵, Asp²⁸] Exendine-4(1-39)-NH₂,
H-Asn-(Glu)₅- du Pro³⁶, Pro³⁷, Pro³⁸ [Trp(O)²⁵,
Asp²⁸] Exendine -4(1-39)-NH₂,
du Pro³⁶, Pro³⁷, Pro³⁸ [Trp(O₂)²⁵, Asp²⁸] Exendine-4 (1-39)-(Lys) 6-NH₂,
H-(Lys)₆,- du Pro³⁶, Pro³⁷, Pro³⁸ [Trp(O₂)²⁵, Asp²⁸] Exendine-4(1-39)-(Lys)₆-NH₂,
H-Asn-(Glu)₅- du Pro³⁶ Pro³⁷ Pro³⁸ [Trp(O₂)²⁵, Asp²⁸] Exendine-4(1-39)-(Lys)₆-NH₂,
H-(Lys)₆- du Pro³⁶ [Met(O)¹⁴, Asp²⁸] Exendine-4(1-39) -Lys₆-NH₂,
du Met(O)¹⁴ Asp²⁸ Pro³⁶, Pro³⁷, Pro³⁸ Exendine-4(1-39)-NH₂, H-(Lys)₆ du Pro³⁶, Pro³⁷, Pro³⁸ [Met(O)¹⁴,
Asp²⁸] Exendine-4(1-39)-NH₂,
H-Asn-(Glu)₅- du Pro³⁶, Pro³⁷, Pro³⁸ [Met(O)¹⁴, Asp²⁸] Exendine-4(1-39)-NH₂,
du Pro³⁶, Pro³⁷, Pro³⁸ [Met(O)¹⁴, Asp²⁸] Exendine-4(1-39) - (Lys) ₆-NH₂,
H-(Lys)₆- du Pro³⁶, Pro³⁷, Pro³⁸ [Met(O)¹⁴ Asp²⁸] Exendine-4(1-39)-(Lys)₆-NH₂,
H-Asn-(Glu)₅ du Pro³⁶, Pro³⁷, Pro³⁸ [Met(O)¹⁴, Asp²⁸] Exendine-4(1-39)-(Lys)₆-NH₂,
H-Lys₆- du Pro³⁶ [Met(O)¹⁴, Trp(O₂)²⁵, Asp²⁸] Exendine-4(1-39)-Lys₆-NH₂,
H- de l'Asp²⁸ Pro³⁶, Pro³⁷, Pro³⁸ [Met(O)¹⁴, Trp(O₂)²⁵] Exendine-4(1-39)-NH₂,
H-(Lys)₆- du Pro³⁶, Pro³⁷, Pro³⁸ [Met(O)¹⁴, Asp²⁸] Exendine-4(1-39)-NH₂,
H-Asn-(Glu)₅- du Pro³⁶, Pro³⁷, Pro³⁸ [Met(O)¹⁴,
Trp(O₂)²⁵, Asp²⁸] Exendine-4(1-39)-NH₂,
du Pro³⁶, Pro³⁷, Pro³⁸ [Met(O)¹⁴, Trp(O₂)²⁵, Asp²⁸] Exendine-4(1-39)-(Lys)₆-NH₂,
H-(Lys)₆- du Pro³⁶, Pro³⁷, Pro³⁸ [Met(O)¹⁴, Trp(O₂)²⁵, Asp²⁸] Exendine-4 (S1-39)-(Lys) ₆-NH₂,
H-Asn-(Glu)₅- du Pro³⁶, Pro³⁷, Pro³⁸ [Met(O)¹⁴,
Trp(O₂)²⁵, Asp²⁸] Exendine-4(1-39)-(Lys)₆-NH₂; ou
du Pro³⁶ [Asp²⁸] Exendine-4(1-39),
du Pro³⁶ [IsoAsp²⁸] Exendine-4(1-39),
du Pro³⁶ [Met(O)¹⁴, Asp²⁸] Exendine-4(1-39),
du Pro³⁶ [Met(O)¹⁴, IsoAsp²⁸] Exendine-4(1-39),
du Pro³⁶ [Trp(O₂)²⁵, Asp²⁸] Exendine-4(1-39),
du Pro³⁶ [Trp(O₂)²⁵, IsoAsp²⁸] Exendine-4(1-39),
du Pro³⁶ [Met(O)¹⁴ Trp(O₂)²⁵, Asp²⁸] Exendine-4 (1-39),
du Pro³⁶ [Met(O)¹⁴ Trp(O₂)²⁵, IsoAsp²⁸ Exendine-4 (1-39),
le groupe -Lys₆-NH₂ étant enchaîné avec le terminus C du dérivé d'Exendine-4 ;
ou un sel ou solvate acceptable du point de vue pharmaceutique de ce dernier.

7. Instrument d'application contenant une ampoule à deux chambres selon l'une quelconque des revendications 1 à 6.

8. Procédé de fabrication et de remplissage d'une ampoule à deux chambres selon l'une quelconque des revendications 1 à 6, **caractérisé en ce que**
a) on munit un cylindre contenant une dérivation d'un bouchon central entre la dérivation et l'extrémité proximale ;
b) on remplit la chambre d'un composant liquide entre le bouchon central et l'extrémité proximale ;
c) on ferme à l'aide d'un bouchon d'extrémité la chambre contenant le composant liquide ;
d) on remplit la chambre d'un composant liquide ou de préférence solide entre le bouchon central et l'extrémité distale du cylindre ; et
e) on ferme l'extrémité distale du cylindre avec une garniture.
